(19) Europäisches Patentamt
European Patent Office
Office européen des brevets

(11) Veröffentlichungsnummer: **0 038 427**
**A1**

# (12) EUROPÄISCHE PATENTANMELDUNG

(21) Anmeldenummer: **81102063.5**

(22) Anmeldetag: **19.03.81**

(51) Int. Cl.³: **C 07 D 311/56**
**C 07 D 407/12, A 01 N 43/16**

(30) Priorität: **01.04.80 DE 3012642**

(43) Veröffentlichungstag der Anmeldung:
**28.10.81 Patentblatt 81/43**

(84) Benannte Vertragsstaaten:
**BE CH DE FR GB IT LI NL**

(71) Anmelder: **BAYER AG**
**Zentralbereich Patente, Marken und Lizenzen**
**D-5090 Leverkusen 1, Bayerwerk(DE)**

(72) Erfinder: **Kranz, Eckart, Dr.**
**Am Acker 9**
**D-5600 Wuppertal 1(DE)**

(72) Erfinder: **Klauke, Erich, Dr.**
**Eichendorffweg 8**
**D-5068 Odenthal(DE)**

(72) Erfinder: **Hammann, Ingeborg, Dr.**
**Belfortstrasse 9**
**D-5000 Köln 1(DE)**

(54) **3-Carbamoyl-4-hydroxy-cumarine, Verfahren zu ihrer Herstellung und ihre Verwendung als Schädlingsbekämpfungsmittel.**

(57) Die vorliegende Erfindung betrifft neue 3-Carbamoyl-4-hydroxy-cumarine der Formel I

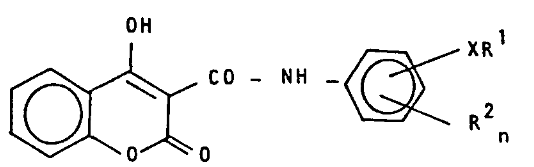

(1)

in welcher X, R¹, R² und n die in der Beschreibung angegebene Bedeutung besitzen. Sie werden erhalten, wenn man 4-Hydroxycumarine mit Isocyanaten in Gegenwart eines Verdünnungsmittels und in Gegenwart einer Base umsetzt. Die neuen 3-Carbamoyl-4-hydroxy-cumarine der Formel (I) weisen starke schädlingsbekämpfende insbesondere insektizide Eigenschaften auf.

EP 0 038 427 A1

Croydon Printing Company Ltd.

BAYER AKTIENGESELLSCHAFT
Zentralbereich
Patente Marken und Lizenzen

5090 Leverkusen-Bayerwerk
Rt/klu.

Ia

3-Carbamoyl-4-hydroxy-cumarine, Verfahren zu ihrer Herstellung und ihre Verwendung als Schädlingsbekämpfungsmittel

Die vorliegende Erfindung betrifft neue 3-Carbamoyl-4-hydroxy-cumarine, ein Verfahren zu ihrer Herstellung und ihre Verwendung als Schädlingsbekämpfungsmittel.

Es ist bereits bekannt geworden, daß bestimmte 3-Carbamoyl-4-hydroxy-cumarine, wie 3-(Halogenphenylcarbamoyl)-4-hydroxy-cumarine, gute insektizide Eigenschaften besitzen (vergleiche DE-OS 26 43 428 und DE-OS 26 43 476). Deren Wirkung ist jedoch, insbesondere bei niedrigen Aufwandmengen, nicht immer ganz befriedigend.

Es wurden neue 3-Carbamoyl-4-hydroxy-cumarine der allgemeinen Formel (I)

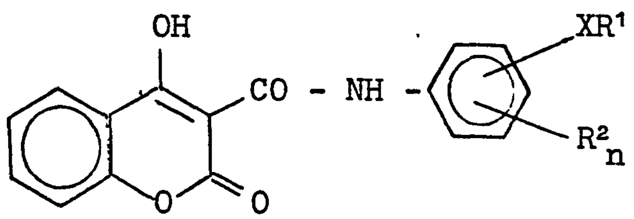
(I)

in welcher

X für Sauerstoff oder Schwefel steht,

$R^1$ für Halogenalkyl, Halogenalkenyl, Halogenalkinyl, Alkyl oder gegebenenfalls substituiertes Phenyl steht,

$R^2$ für Halogen, Alkyl, Halogenalkyl, Nitro oder Cyano steht,

$XR^1$ und $R^2$ gemeinsam in ortho-Stellung zueinander für einen gegebenenfalls substituierten ankondensierten Dioxan-Rest stehen und

n für 0, 1 oder 2 steht,

gefunden.

Weiterhin wurde gefunden, daß man die 3-Carbamoyl-4-hydroxy-cumarine der Formel (I) erhält, wenn man 4-Hydroxy-cumarine der Formel (II)

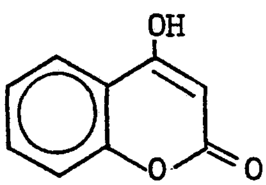

(II)

mit Isocyanaten der Formel (III)

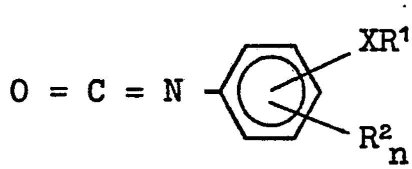

(III)

in welcher

X,$R^1$,$R^2$ und n die oben angegebene Bedeutung haben,

in Gegenwart eines Verdünnungsmittels und in Gegenwart einer Base umsetzt.

Die neuen 3-Carbamoyl-4-hydroxy-cumarine der Formel (I) weisen starke schädlingsbekämpfende insbesondere insektizide Eigenschaften auf. Überraschenderweise zeigen die erfindungsgemäßen Verbindungen dabei eine höhere Wirkung als die bekannten 3-(Halogenphenyl-carbamoyl)-4-hydroxy-cumarine, welche chemisch und wirkungsmäßig naheliegende Verbindungen sind. Die erfindungsgemäßen Wirkstoffe stellen somit eine Bereicherung der Technik dar.

Die erfindungsgemäßen 3-Carbamoyl-4-hydroxy-cumarine sind durch die Formel (I) allgemein definiert. In dieser Formel steht <u>$R^1$</u> vorzugsweise für Halogenalkyl mit 1 bis 2 Kohlenstoff- und bis zu 5 gleichen oder verschiedenen Halogenatomen, wie insbesondere Fluor- und Chloratomen; für Halogenalkenyl und Halogenalkinyl mit jeweils 2 bis 4 Kohlenstoffatomen und 1 bis 5 Halogenatomen, wie insbesondere Fluor- und Chloratomen; für Alkyl mit 1 bis 4 Kohlenstoffatomen sowie für gegebenenfalls durch Halogen und Alkyl mit 1 bis 2 Kohlenstoffatomen substituiertes Phenyl. <u>$R^2$</u> steht vorzugsweise für Halogen; geradkettiges oder verzweigtes Alkyl mit 1 bis 4 Kohlenstoffatomen; Halogenalkyl mit 1 bis 2 Kohlenstoff- und bis zu 5 gleichen oder

verschiedenen Halogenatomen, wie insbesondere Fluor- und Chloratomen; Nitro oder Cyano. $\underline{X}\underline{R}^1$ und $\underline{R}^2$ stehen außerdem gemeinsam in ortho-Stellung zueinander vorzugsweise für einen gegebenenfalls durch Halogen substituierten ankondensierten Dioxan-Rest. X und der Index n haben vorzugsweise die in der Erfindungsdefinition angegebene Bedeutung.

Besonders bevorzugt sind diejenigen Verbindungen der Formel (I), in denen X für Sauerstoff oder Schwefel steht; $\underline{R}^1$ für Trifluormethyl, Difluorchlormethyl, Fluordichlormethyl, Trichlormethyl, Dichlormethyl, Chlormethyl;1,1,2-Trifluor-2-chlor-ethyl, 1,1,2-Trichlorethyl, 1,1-Dibromethyl, 1,1-Dichlorethyl, 1,1,2-Trichlorpropyl, 2-Chlor-prop-2-yl, 1,1,2,3,3,3-Pentafluorpropyl, 1,2,2-Trichlorvinyl, 2,2-Dichlorvinyl, Methyl, Ethyl, Isopropyl oder gegebenenfalls durch Fluor, Chlor und Methyl substituiertes Phenyl steht; $\underline{R}^2$ für Fluor, Chlor, Brom, Jod, Methyl, Ethyl, Isopropyl, Trifluormethyl, Nitro oder Cyano steht; $\underline{X}\underline{R}^1$ und $\underline{R}^2$ gemeinsam in ortho-Stellung zueinander for einen gegebenenfalls durch Fluor substituierten ankondensierten Dioxan-Rest stehen; und der Index n für 0 oder 1 steht.

Verwendet man beispielsweise 4-Hydroxy-cumarin und 4-Trifluormethoxyphenylisocyanat als Ausgangsstoffe, so kann der Reaktionsablauf durch das folgende Formelschema wiedergegeben werden:

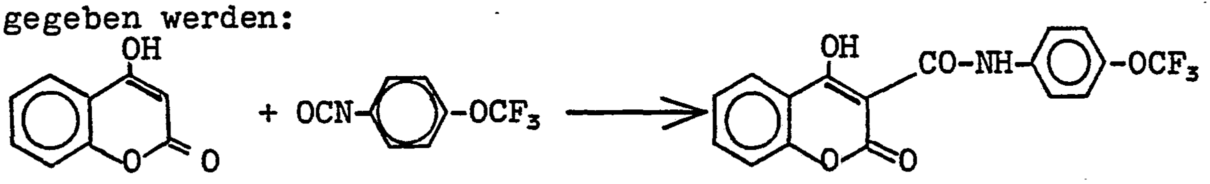

Die für das erfindungsgemäße Verfahren als Ausgangsstoffe benötigten Isocyanate sind durch die Formel (III) allgemein definiert. In dieser Formel stehen X, $\underline{R}^1$, $\underline{R}^2$ und der Index n vorzugsweise für die Reste, die bei der Beschreibung der erfindungsgemäßen Stoffe der Formel (I) bereits für diese Substituenten vorzugsweise genannt wurden.

Die Isocyanate der Formel (III) sind bekannt, bzw. lassen sie sich in allgemein bekannter Art und Weise herstellen.

Das außerdem als Ausgangsstoff zu verwendende 4-Hydroxycumarin ist ebenfalls eine allgemein bekannte Verbindung der organischen Chemie.

Als Verdünnungsmittel kommen für die erfindungsgemäße Umsetzung alle inerten organischen Lösungsmittel infrage. Hierzu gehören vorzugsweise Ketone, wie Aceton und Methylethylketon; Nitrile, wie Acetonitril und Propionitril; Ether, wie Diisopropylether, Dioxan und Tetrahydrofuran; Formamide, wie Dimethylformamid; aliphatische, aromatische und halogenierte Kohlenwasserstoffe, wie Toluol, Xylol, Chlorbenzol, Methylenchlorid und Chloroform; sowie Dimethylsulfoxid.

Die erfindungsgemäße Umsetzung wird in Gegenwart einer Base durchgeführt. Hierzu können alle üblichen organischen und anorganischen Basen eingesetzt werden. Vorzugsweise seien genannt: tertiäre Amine, wie Triethylamin und Dimethylanilin; ferner Pyridin, Alkalimetallhydroxide oder -carbonate, wie Natrium- und Kaliumhydroxid, Natrium- und Kaliumcarbonat oder Natriumhydrogencarbonat; sowie Alkalimetallalkoholate, wie Natriummethylat oder Kalium-tert.-butylat.

Die Reaktionstemperaturen können bei der Durchführung des erfindungsgemäßen Verfahrens in einem größeren Bereich variiert werden. Im allgemeinen arbeitet man zwischen 0 und 150°C, vorzugsweise bei 0 bis 100°C.

Bei der Durchführung des erfindungsgemäßen Verfahrens arbeitet man vorzugsweise in molaren Mengen. Die Isolierung der Verbindungen der Formel (I) erfolgt in allgemein üblicher Weise.

Die Wirkstoffe eignen sich bei guter Pflanzenverträglichkeit und günstiger Warmblütertoxizität zur Bekämpfung von tierischen Schädlingen, insbesondere Insekten, Spinnentieren, die in der Landwirtschaft, in Forsten, im Vorrats- und Materialschutz sowie auf dem Hygienesektor vorkommen.Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.
Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp.,Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.
Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp. Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia podana, Capua reticulana, Choristoneura fumiferana, Clysia ambiguella, Homona magnanima, Tortrix viridana.
Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp.,

Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..
Aus der Ordnung der Arachnida z.B. Scorpio maurus, Latrodectus mactans.
Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff

und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyäthylen-Fettsäure-Ester, Polyoxyäthylen-Fettalkohol-Äther, z.B. Alkylarylpolyglykol-äther, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige und latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die Anwendung der erfindungsgemäßen Wirkstoffe erfolgt in Form ihrer handelsüblichen Formulierungen und/oder den aus diesen Formulierungen bereiteten Anwendungsformen.

Der Wirkstoffgehalt der aus den handelsüblichen Formulierungen bereiteten Anwendungsformen kann in weiten Bereichen variieren. Die Wirkstoffkonzentration der Anwendungsformen kann von 0,000001 bis zu 100 Gew.-% Wirkstoff, vorzugsweise zwischen 0,01 und 10 Gew.-% liegen.
Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Beispiel

Phaedon-Larven-Test

Lösungsmittel: 3 Gewichtsteile Dimethylformamid
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven (Phaedon cochleariae) besetzt, solange die Blätter noch feucht sind.

Nach der gewünschten Zeit wird die Abtötung in % bestimmt. Dabei bedeutet 100 %, daß alle Käfer-Larven abgetötet wurden; 0 % bedeutet, daß keine Käfer-Larven abgetötet wurden.

Bei diesem Test zeigen z.B. die folgenden Verbindungen der Herstellungsbeispiele überlegene Wirksamkeit gegenüber dem Stand der Technik: 1,2,3,4,7,8,10,11 und 12.

Herstellungsbeispiele

Beispiel 1

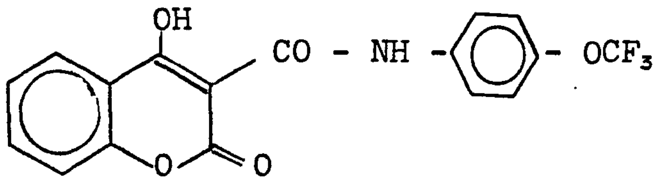

Zu 8,1g (0,05 Mol) 4-Hydroxycumarin in 100 ml Dimethylsulfoxid werden bei Raumtemperatur zunächst 5,1g (0,05 Mol) Triethylamin und danach langsam 10,2g (0,05 Mol) 4-Trifluormethoxyphenylisocyanat zugetropft. Dabei steigt die Temperatur auf 31°C. Man läßt 5 Stunden bei Raumtemperatur nachrühren. Das Reaktionsgemisch wird zu einer Mischung aus 250 ml kaltem Wasser und 15 ml konzentrierter Salzsäure gegeben, wobei ein weißer Niederschlag ausfällt. Man verrührt 30 Minuten unter Kühlung und saugt den Niederschlag ab, wäscht ihn mit saurem Wasser nach und trocknet bei 50°C im Vakuum. Nach Unkristallisation in 100 ml Dioxan erhält man 8,3g (45,6 % der Theorie) 4-Hydroxy-3-(4-trifluormethoxyphenyl-carbamoyl)-cumarin vom Schmelzpunkt 194-195°C.

In analoger Weise können die folgenden Verbindungen der allgemeinen Formel

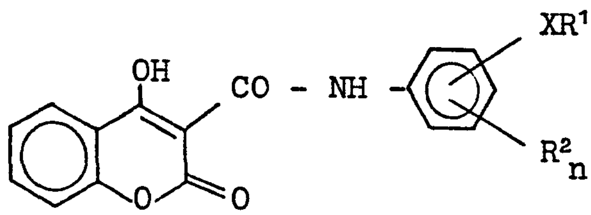

(I)

erhalten werden:

| Bsp. Nr. | $XR^1$ | $R^2_n$ | Schmelzpunkt (°C) |
|---|---|---|---|
| 2 | $4-SCF_3$ | 3-Cl | 201-03 |
| 3 | $4-SCF_2Cl$ | 3-Cl | 206-07 |
| 4 | $4-OCF_2-CHFCl$ | - | 210-11 |
| 5 | $4-SCF_3$ | - | 234-38 |
| 6 | $3,4-CF_2-O-CF_2-O-$ | | 236-42 |
| 7 | $4-OCF_2-CHF-CF_3$ | - | 174-78 |
| 8 | $4-OCF_2Cl$ | - | 189-92 |
| 9 | $4-OCH_3$ | $3-CF_3$ | 285-94 |
| 10 | $4-OCF_3$ | 2-Cl | 182-92 |
| 11 | $3-OCF_3$ | - | 160-65 |
| 12 | $4-OCF_3$ | 3-Cl | 200-04 |
| 13 | 4-O- 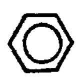 -Cl | $3-CF_3$ | 181-88 |
| 14 | $3,4-O-CF_2-CF_2-O-$ | | 258-63 |

Patentansprüche:

1. 3-Carbamoyl-4-hydroxy-cumarine der allgemeinen Formel (I)

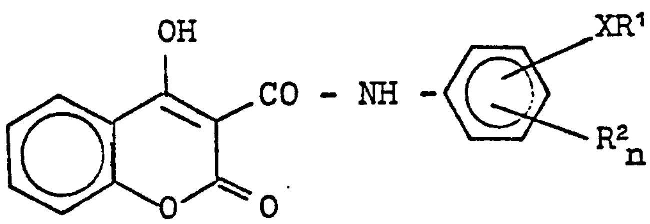

(I)

in welcher

X für Sauerstoff oder Schwefel steht,

$R^1$ für Halogenalkyl, Halogenalkenyl, Halogenalkinyl, Alkyl oder gegebenenfalls substituiertes Phenyl steht,

$R^2$ für Halogen, Alkyl, Halogenalkyl, Nitro oder Cyano steht,

$XR^1$ und $R^2$ gemeinsam in ortho-Stellung zueinander für einen gegebenenfalls substituierten ankondensierten Rest stehen und

n für O, 1 oder 2 steht.

2. Verfahren zur Herstellung der 3-Carbamoyl-4-hydroxy-cumarine der Formel (I) das dadurch gekennzeichnet ist, daß man 4-Hydroxy-cumarine der Formel (II)

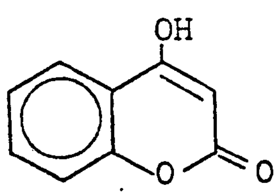

(II)

mit Isocyanaten der Formel (III)

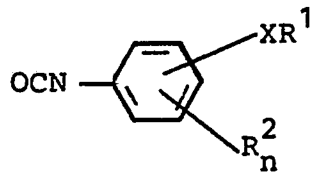

III

in welcher
X, $R^1$, $R^2$ und n die oben angegebene Bedeutung haben,

in Gegenwart eines Verdünnungsmittels und in Gegenwart einer Base umsetzt.

3. Schädlingsbekämpfungsmittel, gekennzeichnet durch einen Gehalt an mindestens einem 3-Carbamoyl-4-hydroxy-cumarin der allgemeinen Formel (I).

4. Verwendung von 3-Carbamoyl-4-hydroxy-cumarinen der Formel (I) zur Bekämpfung von Schädlingen.

5. Verfahren zur Bekämpfung von Schädlingen, dadurch gekennzeichnet, daß man 3-Carbamoyl-4-hydroxy-cumarine der Formel (I) auf Schädlinge und/oder ihren Lebensraum einwirken läßt.

6. Verfahren zur Herstellung von Schädlingsbekämpfungsmitteln, dadurch gekennzeichnet, daß man 3-Carbamoyl-4-hydroxy-cumarine der Formel (I) mit Streckmitteln und/oder oberflächenaktien Mitteln vermischt.

0038427

Europäisches Patentamt

**EUROPÄISCHER RECHERCHENBERICHT**

Nummer der Anmeldung
EP 81 10 2063

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| X | US - A - 3 511 856 (JOHN S.Mc. INTYRE)<br>* Insgesamt *<br>--- | 1-5 |
| X | CHEMICAL ABSTRACTS, Band 81, 1974, Seite 88, Zusammenfassung 86751c, COLUMBUS, OHIO (US)<br>& JP - A - 74 26 427 (HOKKO CHEMICAL INDUSTRY Co. Ltd.; UBE INDUSTRIES Ltd.), 08.03.1974<br>* Insgesamt *<br>--------- | 1-5 |

KLASSIFIKATION DER ANMELDUNG (Int. Cl.³)

C 07 D 311/56
C 07 D 407/12
A 01 N 43/16

RECHERCHIERTE SACHGEBIETE (Int. Cl.³)

C 07 D 311/56
C 07 D 407/12
A 01 N 43/16

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 29.07.1981 | FRANCOIS |